(19)

Europäisches
Patentamt
European
Patent Office
Office européen
des brevets

(11) **EP 3 168 636 A2**

(12) **EUROPEAN PATENT APPLICATION**

(43) Date of publication:
**17.05.2017 Bulletin 2017/20**

(21) Application number: **16198067.7**

(22) Date of filing: **10.11.2016**

(51) Int Cl.:
**G01R 33/56** (2006.01)     **G01R 33/563** (2006.01)
**G01R 33/561** (2006.01)     **G01R 33/567** (2006.01)
**A61B 5/055** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **10.11.2015   KR 20150157487
07.07.2016   KR 20160086408**

(71) Applicants:
• **Samsung Electronics Co., Ltd.
Gyeonggi-do 16677 (KR)**
• **Korea Advanced Institute of Science and
Technology
Daejeon (KR)**

(72) Inventors:
• **KIM, Young-beom
Gyeonggi-do (KR)**
• **PARK, Hyun-wook
Daejeon (KR)**
• **KIM, Dong-chan
Daejeon (KR)**
• **SEO, Hyun-seok
Daejeon (KR)**
• **KWON, Ki-nam
Daejeon (KR)**
• **CHO, Jae-jin
Daejeon (KR)**

(74) Representative: **D'Halleweyn, Nele Veerle Trees
Gertrudis et al
Arnold & Siedsma
Bezuidenhoutseweg 57
2594 AC The Hague (NL)**

(54)     **GRADIENT ECHO MRI USING VELOCITY-SELECTIVE EXCITATION**

(57)     An MRI apparatus includes a controller configured to acquire, based on a gradient echo sequence, an MR signal from an object; and an image processor configured to acquire a first image corresponding to a time point when the MR signal has a largest phase variation, acquire a second image corresponding to a time point when the MR signal has a smallest phase variation, and obtain an arterial image including an artery of the object by using the first image and the second image.

FIG. 9

**Description**

CROSS-REFERENCE TO RELATED APPLICATIONS

This application claims priority from Korean Patent Application No. 10-2015-0157487, filed on November 10, 2015, and Korean Patent Application No. 10-2016-0086408, filed on July 7, 2016, in the Korean Intellectual Property Office, the disclosures of which are incorporated herein in their entireties by reference.

BACKGROUND

**1. Field**

The present disclosure relates to magnetic resonance imaging (MRI) apparatuses and methods, and more particularly, to MRI apparatuses and methods of capturing MR images by using velocity-selective excitation and a gradient echo sequence.

**2. Description of the Related Art**

A magnetic resonance imaging (MRI) apparatus uses a magnetic field to capture an image of a target object. The MRI apparatus is widely used for accurate disease diagnosis because stereoscopic images of bones, lumbar discs, joints, nerve ligaments, the heart, etc. can be obtained at desired angles.

In order to capture an MR image, an MRI apparatus applies a radio frequency (RF) signal to an object and acquires an MR signal emitted from the object in response to the applied RF signal. To obtain a high quality MR image, the MRI apparatus may reconstruct a motion-corrected MR image from an acquired MR signal by correcting for artifacts caused by motion of an object that has occurred during an MRI scan.

During an MRI scan of the lower half of the body, a related art MRI apparatus obtains an MR image based on the fact that a velocity of blood flow varies based on a heart rate and the blood flow generates a signal having a higher intensity than static tissue.

In order to obtain information about a heart rate of an object, related art MRI requires the use of electrocardiogram (ECG) gating equipment and application of repetitive RF pulses. According to this approach, a specific absorption rate (SAR) may be increased, and an MRI scan may take a little longer.

SUMMARY

Provided are magnetic resonance imaging (MRI) apparatuses and methods, whereby a specific absorption rate (SAR) and scanning time may be reduced by capturing an MR image by using velocity-selective excitation and a gradient echo sequence.

Provided are MRI apparatuses and methods, whereby field homogeneity may be improved and accordingly a high quality image may be obtained by capturing an MR image by using velocity-selective excitation and a gradient echo sequence and without using electrocardiogram (ECG) gating or a saturation pulse.

Additional aspects will be set forth in part in the description which follows and, in part, will be apparent from the description, or may be learned by practice of the presented embodiments.

According to an aspect of an embodiment, an MRI apparatus includes a controller configured to acquire, based on a gradient echo sequence, an MR signal from an object; and an image processor configured to acquire a first image corresponding to a time point when the MR signal has a largest phase variation, acquire a second image corresponding to a time point when the MR signal has a smallest phase variation, and obtain an arterial image including an artery of the object by using the first image and the second image.

According to an aspect of another embodiment, a method of obtaining an MR image includes acquiring an MR signal from an object based on a gradient echo sequence; acquiring a first image corresponding to a time point when the MR signal has a largest phase variation and a second image corresponding to a time point when the MR signal has a smallest phase variation; and obtaining an arterial image including an artery of the object by using the first image and the second image.

According to the embodiments, an MR image is captured by using velocity-selective excitation and a gradient echo sequence, thereby reducing a SAR and a scan time.

According to the embodiments, an MR image is obtained by using velocity-selective excitation and a gradient echo sequence and without using ECG gating and a saturation pulse. Thus, field homogeneity may be improved, and accordingly, a high quality image may be obtained.

BRIEF DESCRIPTION OF THE DRAWINGS

These and/or other aspects will become apparent and more readily appreciated from the following description of the embodiments, taken in conjunction with the accompanying drawings in which:

FIG. 1 is a block diagram of a magnetic resonance imaging (MRI) apparatus according to an embodiment;
FIG. 2 is a diagram for explaining a process, performed by an MRI apparatus, of obtaining an MR image by using electrocardiogram (ECG)-gated two-dimensional time of flight (2D-TOF), according to an embodiment;
FIG. 3 is a diagram for explaining a process, performed by an MRI apparatus, of obtaining an MR image of an object by using single-shot balanced steady-state free precession (bSSFP), according to an embodiment;
FIG. 4A illustrates a pulse sequence used by an MRI apparatus according to an embodiment;
FIG. 4B illustrates a change in an MR signal acquired by an MRI apparatus from an object at a plurality of time points, according to an embodiment;
FIG. 5A is a graph of a phase variation of an MR signal, which is acquired by an MRI apparatus, with respect to a heart rate, according to an embodiment;
FIG. 5B is a graph of intensity of an image signal with respect to a velocity of blood flow according to an embodiment;
FIG. 6 is a diagram for explaining a process, performed by an MRI apparatus, of reconstructing an undersampled image, according to an embodiment;
FIG. 7 illustrates a process, performed by an MRI apparatus, of obtaining an image including an artery, according to an embodiment;
FIG. 8 illustrates an image obtained by an MRI apparatus according to an embodiment;
FIG. 9 is a flowchart of a method, performed by an MRI apparatus, of obtaining an MR image according to an embodiment; and
FIG. 10 is a schematic diagram of an MRI system.

DETAILED DESCRIPTION

The present specification describes principles of the present disclosure and sets forth embodiments thereof to clarify the scope of the present disclosure and to allow those of ordinary skill in the art to implement the embodiments. The present embodiments may have different forms and should not be construed as being limited to the descriptions set forth herein.

Like reference numerals refer to like elements throughout. The present specification does not describe all components in the embodiments, and common knowledge in the art or the same descriptions of the embodiments will be omitted below. The term "part" or "portion" may be implemented using hardware or software, and according to embodiments, one "part" or "portion" may be formed as a single unit or element or include a plurality of units or elements. Expressions such as "at least one of," when preceding a list of elements, modify the entire list of elements and do not modify the individual elements of the list. Hereinafter, the principles and embodiments of the present disclosure will be described in detail with reference to the accompanying drawings.

In the present specification, an "image" may include a medical image obtained by a magnetic resonance imaging (MRI) apparatus, a computed tomography (CT) apparatus, an ultrasound imaging apparatus, an X-ray apparatus, or another medical imaging apparatus.

Furthermore, in the present specification, an "object" may be a target to be imaged and include a human, an animal, or a part of a human or animal. For example, the object may include a body part (an organ) or a phantom.

An MRI system acquires an MR signal and reconstructs the acquired MR signal into an image. The MR signal denotes a radio frequency (RF) signal emitted from the object.

In the MRI system, a main magnet creates a static magnetic field to align a magnetic dipole moment of a specific atomic nucleus of the object placed in the static magnetic field along a direction of the static magnetic field. A gradient coil may generate a gradient magnetic field by applying a gradient signal to a static magnetic field and induce resonance frequencies differently according to each region of the object.

An RF coil may emit an RF signal to match a resonance frequency of a region of the object whose image is to be acquired. Furthermore, when gradient magnetic fields are applied, the RF coil may receive MR signals having different resonance frequencies emitted from a plurality of regions of the object. Though this process, the MRI system may obtain an image from an MR signal by using an image reconstruction technique.

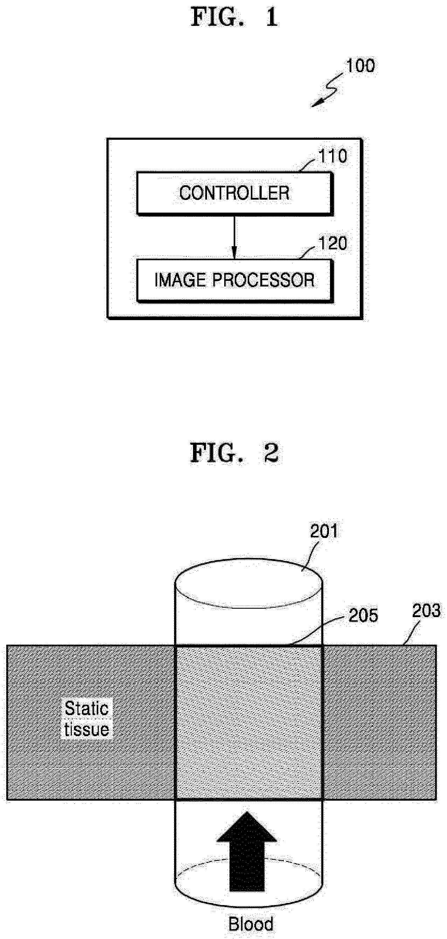

FIG. 1 is a block diagram of an MRI apparatus 100 according to an embodiment.

The MRI apparatus 100 may be an apparatus for obtaining an MR image by performing an MRI scan on an object. Furthermore, the MRI apparatus 100 may be an apparatus for obtaining an MR image by processing MR data acquired by performing an MRI scan on the object.

For example, the MRI apparatus 100 may be an apparatus configured to apply RF pulses to the object via a plurality of channel coils in an RF multi-coil (not shown) and reconstruct MR images based on MR signals acquired via the plurality of channel coils.

Furthermore, the MRI apparatus 100 may be a server apparatus configured to provide a pulse sequence to be applied to the object and reconstruct an MR image based on an MR signal acquired with the pulse sequence. In this case, the server apparatus may be a medical server apparatus in a hospital where an MRI scan is performed on a patient or another hospital.

Referring to FIG. 1, the MRI apparatus 100 may include a controller 110, e.g., a microprocessor, and an image processor 120.

The controller 110 may acquire an MR signal from an object based on a pulse sequence. The controller 110 may control reception of an MR signal by the MRI apparatus 100 according to a pulse sequence stored in a memory (not shown) of the MRI apparatus 100 or a pulse sequence received from an external device (not shown).

According to an embodiment, the controller 110 may acquire, from an object, an MR signal produced based on a pulse sequence, e.g., a gradient echo sequence. In this case, the gradient echo sequence may be a pulse sequence for acquiring k-space data by using a radial trajectory.

According to an embodiment, a gradient echo sequence may include a velocity-selective excitation block. The velocity-selective excitation block may include a velocity encoding gradient (VENC). The VENC may be a gradient for generating a phase variation of an MR signal corresponding to a velocity of blood flow in the object.

According to an embodiment, the velocity-selective excitation block may sequentially include a first RF pulse having a first flip angle, a VENC, and a second RF pulse having a second flip angle with the same magnitude as but a different direction than the first flip angle.

The controller 110 may acquire an MR signal having a phase variation corresponding to a velocity of blood flow included in the object by using a velocity-selective excitation block. Furthermore, the controller 110 may acquire a signal corresponding to an electrical activity of the heart based on the phase variation of the MR signal.

The image processor 120 may reconstruct an image of the object based on the MR signal acquired by the controller 110 from the object.

The image processor 120 may acquire a first image corresponding to a time point when an MR signal has a largest phase variation and a second image corresponding to a time point when the MR signal has a smallest phase variation. In this case, the phase variation may correspond to a velocity of blood flow in the object. The first and second images may be a plurality of undersampled images obtained by the image processor 120.

The image processor 120 may obtain an arterial image including an artery of the object by using the first and second images.

The image processor 120 may acquire k-space data based on an MR signal. For example, the image processor 120 may acquire pieces of k-space data based on MR signals along a radial trajectory.

The image processor 120 may reconstruct, based on the acquired pieces of k-space data, a plurality of undersampled images corresponding to different time points. Each of the plurality of undersampled images may be obtained based on a window containing a plurality of views. Each of the plurality of views corresponds to k-space data acquired for each golden angle along a radial trajectory. Furthermore, the image processor 120 may use another reconstruction method to acquire k-space data.

The image processor 120 may determine first and second images from among the plurality of undersampled images, based on signal intensities of an artery in the plurality of undersampled images.

The image processor 120 may obtain an arterial image based on a difference between intensities of image signals respectively corresponding to the first and second images.

The image processor 120 may acquire a plurality of first images and a plurality of second images. The image processor 120 may obtain a first composite image by combining the acquired plurality of first images and a second composite image by combining the acquired plurality of second images.

According to an embodiment, the first and second images may respectively correspond to systole and diastole phases of the heart of the object. The first and second images may each include an artery and a vein of the object.

The MRI apparatus 100 may further include a display (not shown). The display may provide data received from the controller 110 and the image processor 120.

The display may display a pulse sequence controlled by the controller 110. The pulse sequence may include a gradient echo sequence.

Furthermore, the display may display image data acquired by the image processor 120. For example, the display may display at least one of an undersampled image, a first image, a second image, and an arterial image.

According to an embodiment, the MRI apparatus 100 enables high quality imaging in a relatively short scan time and may be used for performing angiography that examines blood vessels in the object including arteries and veins.

FIG. 2 is a diagram for explaining a process, performed by the MRI apparatus 100, of obtaining an MR image by using electrocardiogram (ECG)-gated two-dimensional time of flight (2D-TOF), according to an embodiment.

In the related art, ECG-gated 2D-TOF has mainly been used to perform MR angiography on a lower half of the body. A 2D TOF method takes advantage of the fact that a velocity of blood flow in a region being imaged varies based on a heart rate while other tissues remain stationary.

Referring to FIG. 2, a color indicated in a 2D MR image may correspond to a magnitude of an image signal. In other words, a dark portion of the 2D MR image may correspond to an image signal having a small magnitude while a bright portion thereof may correspond to an image signal having a large magnitude.

For example, if a static tissue 203 is subjected to repeated RF pulses and spoiling gradients, signal intensity of the static tissue 203 in a reconstructed image may be reduced. When an RF pulse and a spoiling gradient are repeatedly applied across a blood vessel 201, and thus, to a new blood flow therein, the blood vessel 201 may have a higher signal intensity than the static tissue 203. In detail, an image signal from blood flow 205 within the blood vessel 201 may have a higher intensity than an image signal from the static tissue 203. In other words, a portion representing the blood flow 205 may be indicated in a brighter color than a portion representing the static tissue 203.

When the MRI apparatus 100 uses the related art ECG-gated 2D-TOF in this manner, repeated application of RF pulses may increase a specific absorption rate (SAR) and a scan time.

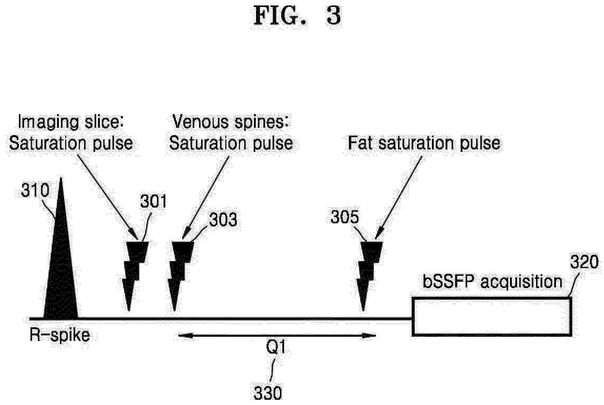

FIG. 3 is a diagram for explaining a process, performed by the MRI apparatus 100, of obtaining an MR image of an object by using single-shot balanced steady-state free precession (bSSFP), according to an embodiment.

Referring to FIG. 3, Quiescent-Interval Single-Shot (QISS) is an image acquisition technique using bSSFP. According to the QISS, following an R-wave 310, the MRI apparatus 100 may saturate an imaging slice and a venous signal by using saturation pulses. When an RF pulse is applied before a spin of an atomic nucleus recovers the longitudinal magnetization, an MR signal produced by the atomic nucleus may be saturated.

After the R-wave 310, the MRI apparatus 100 may apply an imaging slice saturation pulse 301 in order to saturate MR signals within an imaging slice. After applying the imaging slice saturation pulse 301, the MRI apparatus 100 may apply a venous saturation pulse 303 in order to saturate an MR signal from a vein. After the time QI required for a new blood to flow into the imaging slice has elapsed from the time when the venous saturation pulse 303 is applied, the MRI apparatus 100 may saturate an MR signal from fat by using a fat saturation pulse 305. Thereafter, the MRI apparatus 100 may acquire an image by using a bSSFP technique. The MRI apparatus 100 may acquire a slice by using the bSSFP technique based on a bSSFP acquisition pulse sequence 320.

According to the bSSFP technique, the MRI apparatus 200 may acquire a slice every one or two R-waves 310. The bSSFP technique may reduce the time required for image acquisition. However, since the bSSFP technique necessarily requires ECG gating and the use of a plurality of saturation pulses (e.g., 301, 303, and 305), the quality of an image is significantly affected by the performance of the plurality of saturation pulses and field homogeneity.

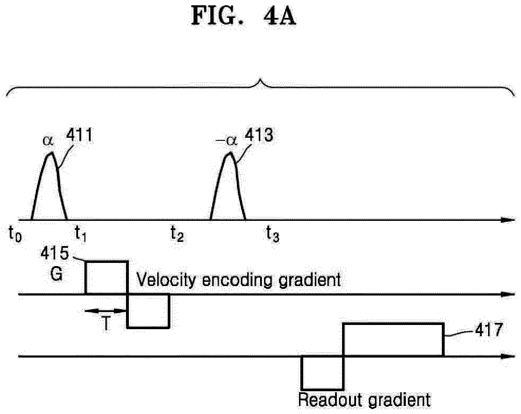

FIG. 4A illustrates a pulse sequence 400 used by the MRI apparatus 100 according to an embodiment.

Referring to FIG. 4A, the pulse sequence 400 may include a gradient echo sequence. The MRI apparatus 100 may acquire an MR signal from an object based on the pulse sequence 400. The pulse sequence 400 may be used to acquire k-space data along a radial trajectory.

The pulse sequence 400 may include a velocity-selective excitation block 410. Furthermore, the pulse sequence 400 may include a readout gradient 417. For convenience of explanation, a slice-selective gradient and a refocusing gradient in the pulse sequence 400 are not shown in FIG. 4A.

According to an embodiment, the velocity-selective excitation block 410 may include a first RF pulse 411, a VENC 415, and a second RF pulse 413. The MRI apparatus 100 may acquire an MR signal having a phase variation corresponding to a velocity by using the velocity-selective excitation block 410. For example, the MRI apparatus 100 may acquire an MR signal having a phase variation corresponding to blood flow in the object by using the velocity-selective excitation block 410.

In the velocity-selective excitation block 410, the first RF pulse 411 may have a first flip angle. Furthermore, the second RF pulse 413 may have a second flip angle. Referring to FIG. 4A, the first flip angle and the second flip angle may be $\alpha$ and $-\alpha$, respectively. In other words, the second RF pulse 413 may have the second flip angle with the same magnitude as but a different direction than the first flip angle of the first RF pulse 411.

The VENC 415 in the velocity-selective excitation 410 may be a gradient for generating a phase variation of an MR signal corresponding to a velocity of blood flow in the object. In FIG. 4A, G and T may respectively denote a magnitude of the VENC 415 and a time interval during which the VENC 415 is applied. The VENC 415 (cm/sec) may represent a maximum measurable velocity of movement. Equation (1) below represents a relationship between a VENC and a strength of a gradient magnetic field:

$$VENC = \pi / 2\gamma \int_{t} G(\tau)\tau d\tau$$

$$\dots (1)$$

where G is a strength of a gradient magnetic field with respect to time and $\gamma$ is a gyromagnetic ratio.

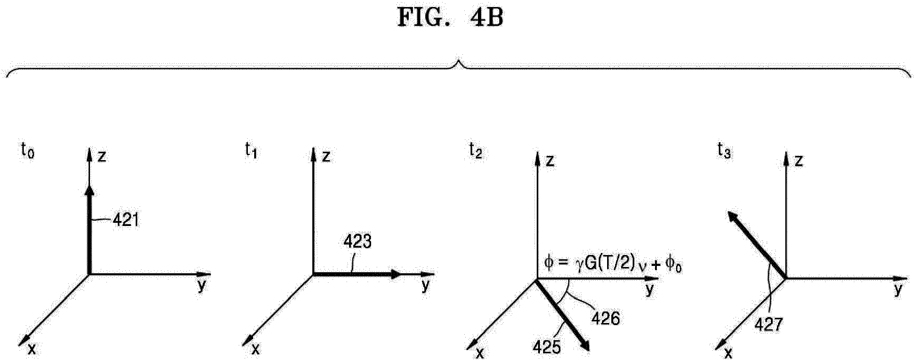

FIG. 4B illustrates a change in an MR signal acquired by the MRI apparatus 100 from an object at a plurality of time points, according to an embodiment.

In detail, FIG. 4B illustrates phases of spins in the object, which are acquired by the MRI apparatus 100 at a plurality of time points $t_0$ through $t_3$. The phases of spins may change based on the first RF pulse (411 of FIG. 4A), the VENC (415 of FIG. 4A), and the second RF pulse (413 of FIG. 4A) included in the velocity-selective excitation block (410 of FIG. 4A).

For example, the time points $t_0$ through $t_3$ may respectively correspond to time points $t_0$ through $t_3$ shown in FIG. 4A. A change in phases 421, 423, 425, and 427 of spins respectively acquired at the time points $t_0$ through $t_3$ will now be described.

The phase 421 of a spin at time point $t_0$ may be oriented toward a z-axis.

After the MRI apparatus 100 applies the first RF pulse 411 to the object, the phase 423 of a spin at time point $t_1$ may be oriented in a direction perpendicular to the z-axis.

A phase 425 of a spin at time point $t_2$, following application of the VENC 415 to the object by the MRI apparatus 100, may be proportional to a velocity at which an atom in the object moves.

Equation (2) below represents a phase of a spin at time point $t_2$ following application of a VENC to an object:

$$\phi = \gamma G (T/2)^2 v + \phi_0 \qquad \dots (2)$$

where v is a velocity at which an atom moves, $\Phi_0$ is a phase value generated due to field inhomogeneity, and G and T are respectively a magnitude of the VENC and a time interval during which the VENC is applied.

The phase of a spin defined by Equation (2) may correspond to a magnitude of an MR signal acquired by the MRI apparatus 100. In other words, a magnitude of an MR signal acquired by the MRI apparatus 100 with a pulse sequence may be determined based on the velocity of an atom.

Furthermore, the magnitude of the MR signal may be determined based on field inhomogeneity at a given point. Thus, the magnitude of the MR signal may be determined based on a velocity of an atom and field inhomogeneity at the given point. For example, an image signal may not be acquired from a point where the velocity of an atom is zero (0) and field inhomogeneity does not exist.

After the MRI apparatus 100 applies the second RF pulse 413 to the object, a phase 427 of a spin at time point $t_3$ may change.

The MRI apparatus 100 may acquire an MR signal that changes based on the phase 427 of the spin. For convenience of explanation, it is assumed hereinafter that a phase of an MR signal corresponds to the phase 427 of the spin.

A phase of an MR signal may be proportional to a velocity at which an atom in an object moves. For example, a phase of an MR signal may be proportional to a velocity of blood flow in the object. The MRI apparatus 100 may reconstruct an image corresponding to a velocity of blood flow in the object based on an MR signal from the blood flow in the object.

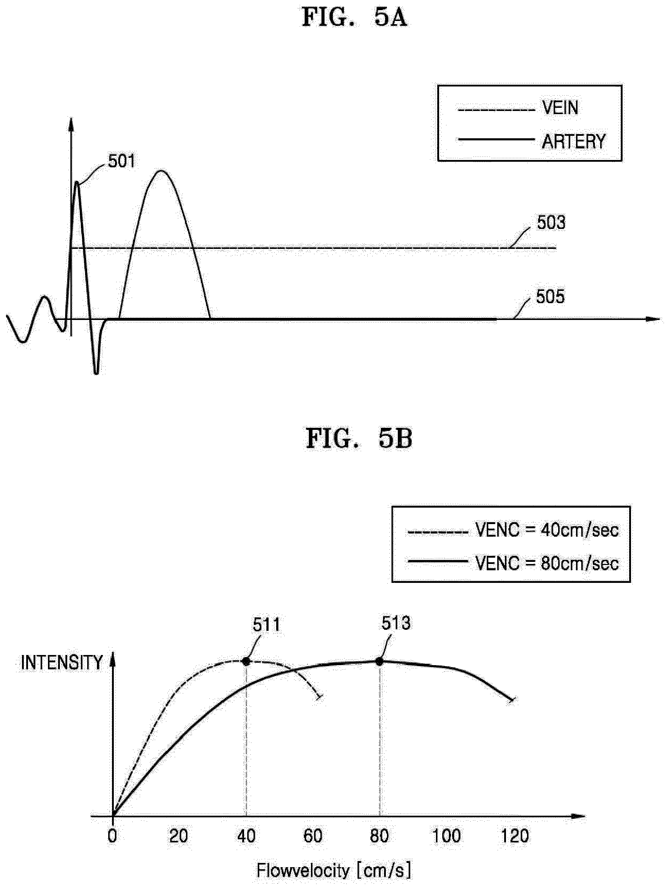

FIG. 5A is a graph of a phase variation of an MR signal, which is acquired by the MRI apparatus 100, with respect to a heart rate, according to an embodiment.

In the graph of FIG. 5A, the ordinate and abscissa respectively denote a phase variation of an MR signal and time. The phase variation of the MR signal may be a phase variation of a spin generated by applying a VENC. After an R-wave 501 occurring due to a heartbeat of an object, a phase variation 503 of an MR signal from blood flow in a vein may not change over time. A phase variation 505 of an MR signal from blood flow in an artery may change over time. This is due to biological characteristics in which a velocity of blood flow in arteries changes depending on a heart rate while velocities of blood flow in veins and other areas remain constant regardless of the heart rate. Although not shown in FIG. 5, a phase offset of a spin may occur as a result of field inhomogeneity. The phase offset may be a phase value of an MR signal, which does not change over time.

FIG. 5B is a graph of intensity of an image signal with respect to a blood flow velocity according to an embodiment.

An image signal may be reconstructed based on a phase of an MR signal, which is proportional to a velocity of blood flow in an object. An intensity of the reconstructed image signal may correspond to the velocity of blood flow in the object. For example, the intensity of the reconstructed MR signal may increase as the velocity of blood flow in the object increases.

In addition, the velocity of blood flow in the object may be measured based on a VENC. The MRI apparatus 100 may determine a maximum velocity of blood flow in the object, which may be measured based on the VENC. The MRI apparatus 100 may vary a portion that is to be depicted clearly in the reconstructed image according to different determined maximum measurable velocities.

Referring to FIG. 5B, if a value of VENC in Equation (1) above is 40 cm/sec, an image signal that can be acquired by reconstructing an MR signal may have a highest intensity when a blood flow velocity is approximately 40 m/sec. Furthermore, if the value of VENC in Equation (1) is 80 cm/sec, an image signal that can be acquired by reconstructing an MR signal may have a highest intensity when the blood flow velocity is approximately 80 m/sec.

The MRI apparatus 100 may set a VENC in Equation (1) to a plurality of values. The MRI apparatus 100 may apply a different VENC according to each of the plurality of values.

The MRI apparatus 100 may reconstruct an image from MR signals acquired based on a plurality of VENCs. Furthermore, the MRI apparatus 100 may respectively reconstruct images based on the plurality of VENCs and combine the reconstructed images to thereby generate a composite image.

The MRI apparatus 100 may reconstruct an image clearly showing all blood vessels with different blood flow velocities by reconstructing images based on different VENCs.

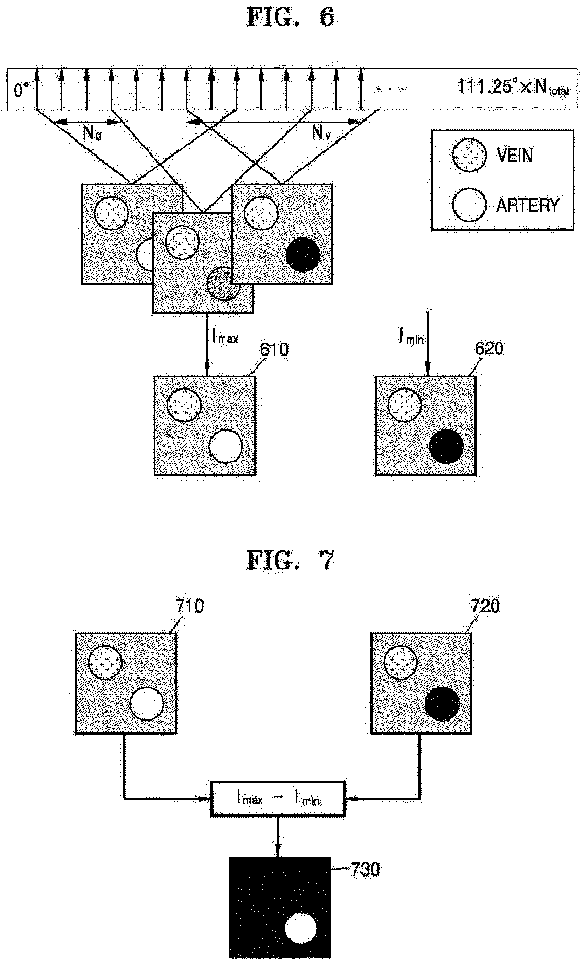

FIG. 6 is a diagram for explaining a process, performed by the MRI apparatus 100, of reconstructing an undersampled image, according to an embodiment.

The MRI apparatus 100 may acquire k-space data based on an MR signal. According to an embodiment, the MRI apparatus 100 may acquire k-space data using a radial trajectory with an increment of a golden angle of 111.25°. The MRI apparatus 100 may obtain, based on k-space data, a plurality of undersampled images respectively corresponding to different time points. In detail, the MRI apparatus 100 may obtain the plurality of undersampled images based on a sliding window reconstruction scheme. According to the sliding window reconstruction scheme, each of the plurality of undersampled images may be obtained based on a window containing a plurality of views. Each of the plurality of views corresponds to k-space data acquired for each golden angle along a radial trajectory.

In FIG. 6, $N_{total}$ denotes a total number of acquired views, and $N_v$ denotes the number of views necessary to reconstruct one undersampled image. In other words, one window may contain a number $N_v$ of views. Ng is an interval between first views in each window. Thus, an undersampled image may be obtained at a time interval corresponding to an interval of $N_g$ views.

Referring to FIG. 6, the MRI apparatus 100 may obtain a plurality of undersampled images 601 respectively corresponding to a plurality of windows.

The undersampled images 601 may correspond to different time points in a cardiac cycle. Referring to FIG. 6, an intensity of an image signal from an artery may vary according to a heart rate. Furthermore, an image signal from a vein may have the same intensity regardless of the heart rate. In the undersampled images 601, intensities of image signals from the artery may vary over the cardiac cycle.

The MRI apparatus 100 may acquire first and second images 610 and 620 based on signal intensities of the artery in the undersampled images 601. For example, image signals from the artery may be extracted by masking signals from a portion corresponding to the artery in the undersampled images 601.

The MRI apparatus 100 may determine, from among the undersampled images 601, the first image 610 corresponding to a time point when an MR signal has a largest phase variation and the second image 620 corresponding to a time point when the MR signal has a smallest phase variation. The MRI apparatus 100 may determine a plurality of first images 610 and a plurality of second images 620 therefrom.

In detail, the first image 610 may correspond to a time point when an MR signal from the artery has a largest phase variation. Furthermore, the first image 610 may correspond to a systole phase of the heart during which a blood flow velocity of the artery is highest.

The second image 620 may correspond to a time point when an MR signal from the artery has a smallest phase variation. Furthermore, the second image 620 may correspond to a diastole phase of the heart during which a blood flow velocity of the artery is lowest.

In addition, according to an embodiment, the MRI apparatus 100 may determine a cardiac cycle based on a change in magnitude of image signals from the artery in the undersampled images 601. Furthermore, the MRI apparatus 100 may acquire a signal corresponding to an electrical activity of the heart based on the change in magnitude of the image signals from the artery in the undersampled images 601.

FIG. 7 illustrates a process, performed by the MRI apparatus 100, of obtaining an image including an artery, according to an embodiment.

The MRI apparatus 100 may determine a first image 710 and a second image 720 from among a plurality of undersampled images acquired using a radial trajectory. The first image 710 may correspond to a time point when an MR signal has a largest phase variation. Furthermore, the second image may correspond to a time point when the MR signal has a smallest phase variation.

The MRI apparatus 100 may obtain an image 730 showing only an artery based on a difference between intensities of signals in the first and second images 710 and 720.

According to an embodiment, the MRI apparatus 100 may generate a first composite image by combining a plurality of first images 710 and a second composite image by combining a plurality of second images 720.

The MRI apparatus 100 may obtain an image showing only an artery based on a difference between intensities of the first and second composite images.

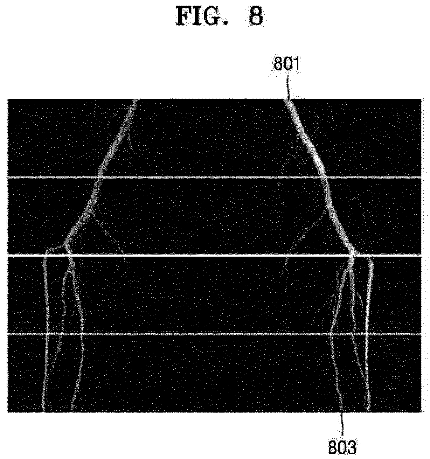

FIG. 8 illustrates an image obtained by the MRI apparatus 100 according to an embodiment

In detail, FIG. 8 shows an image obtained by performing MRI on an object including a lower half of the body

The following parameters were used in an experiment performed with the MRI apparatus 100:

TR/TE = 9ms/15ms, flip angle = 45°
Slice thickness = 3mm (0.7mm overlap)
Effective slice thickness = 2.7 mm
$N_{total}$ / $N_v$ / Ng = 400 / 40 / 4.
Acquisition time per slice= 3.4s.
Total number of slices = 200

Referring to FIG. 8, it can be seen that the MRI apparatus 100 obtained a high quality image by using the characteristics in which only a velocity of blood flow in an artery varies while velocities of blood flow from a vein and other areas remain constant.

As is apparent from FIG. 8, an image signal from an arterial blood vessel 801 having a high blood flow velocity has a high intensity. On the other hand, an image signal from the surrounding blood vessel 803 has a lower intensity than that of the image signal from the arterial blood vessel 801.

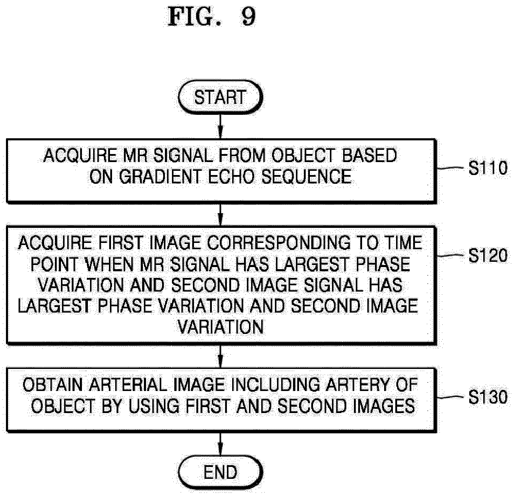

FIG. 9 is a flowchart of a method, performed by the MRI apparatus 100, of obtaining an MR image according to an embodiment.

The MRI apparatus 100 may acquire an MR signal from an object based on a gradient echo sequence (S110). According to an embodiment, the gradient echo sequence may include a velocity-selective excitation block.

The MRI apparatus 100 may acquire a first image corresponding to a time point when an MR signal has a largest phase variation and a second image corresponding to a time point when the MR signal has a smallest phase variation (S120). According to an embodiment, the first image and the second image may respectively correspond to a systole phase and a diastole phase of the heart of the object.

The MRI apparatus 100 may obtain an arterial image including an artery of the object by using the first and second images (S130). According to an embodiment, the arterial image may be obtained based on a difference between intensities of image signals in the first and second images.

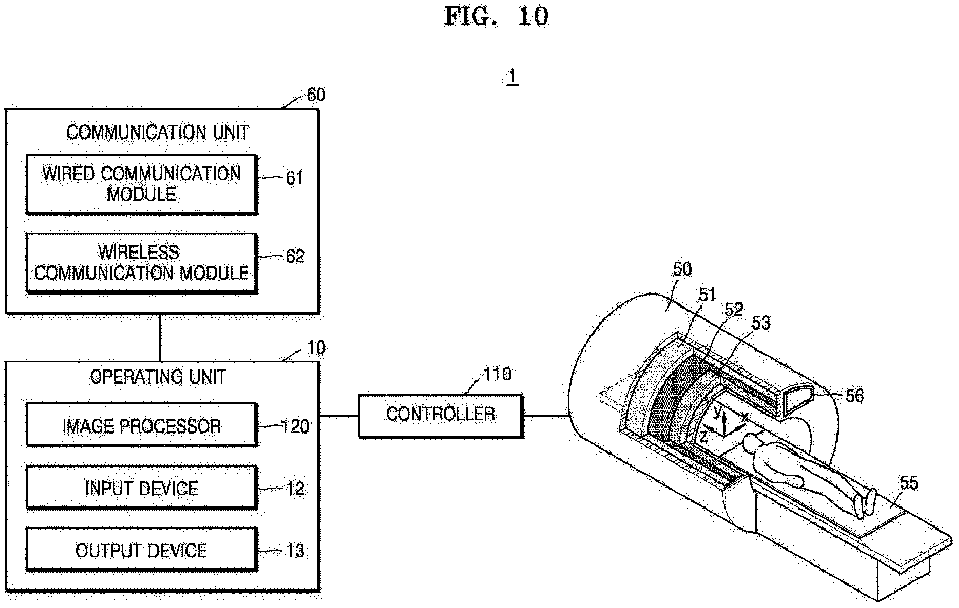

FIG. 10 is a schematic diagram of an MRI system 1 which may include the MRI apparatus 100.

Referring to FIG. 10, the MRI system 1 may include an operating unit 10, a controller 110, and a scanner 50. The controller 110 may be independently separated from the operating unit 10 and the scanner 50. Furthermore, the controller 110 may be separated into a plurality of subcomponents and incorporated into the operating unit 10 and the scanner 50 in the MRI system 1. Operations of the components in the MRI system 1 will now be described in detail.

The scanner 50 may be formed to have a cylindrical shape (e.g., a shape of a bore) having an empty inner space into which an object may be inserted. A static magnetic field and a gradient magnetic field are created in the inner space of the scanner 50, and an RF signal is emitted toward the inner space.

The scanner 50 may include a static magnetic field generator 51, a gradient magnetic field generator 52, an RF coil unit 53, a table 55, and a display 56. The static magnetic field generator 51 creates a static magnetic field for aligning magnetic dipole moments of atomic nuclei of the object in a direction of the static magnetic field. The static magnetic field generator 51 may be formed as a permanent magnet or superconducting magnet using a cooling coil.

The gradient magnetic field generator 52 is connected to the controller 110 and generates a gradient magnetic field by applying a gradient to a static magnetic field in response to a control signal received from the controller 110. The gradient magnetic field generator 52 includes X, Y, and Z coils for generating gradient magnetic fields in X-, Y-, and Z-axis directions crossing each other at right angles and generates a gradient signal according to a position of a region being imaged so as to differently induce resonance frequencies according to regions of the object.

The RF coil unit 53 may emit an RF signal toward the object in response to a control signal received from the controller 110 and receive an MR signal emitted from the object. In detail, the RF coil unit 53 may transmit, toward atomic nuclei of the object having precessional motion, an RF signal having the same frequency as that of the precessional motion, stop transmitting the RF signal, and then receive an MR signal emitted from the object.

The RF coil unit 53 may be formed as a transmitting RF coil for generating an electromagnetic wave having an RF corresponding to the type of an atomic nucleus, a receiving RF coil for receiving an electromagnetic wave emitted from an atomic nucleus, or one transmitting/receiving RF coil serving both functions of the transmitting RF coil and receiving RF coil. Furthermore, in addition to the RF coil unit 53, a separate coil may be attached to the object. Examples of the separate coil may include a head coil, a spine coil, a torso coil, and a knee coil according to a region being imaged or to which the separate coil is attached.

The display 56 may be disposed outside and/or inside the scanner 50. The display 56 is also controlled by the controller 110 to provide a user or the object with information related to medical imaging.

Furthermore, the scanner 50 may include an object monitoring information acquisition unit (not shown) configured to acquire and transmit monitoring information about a state of the object. For example, the object monitoring information acquisition unit may acquire monitoring information related to the object from a camera (not shown) for capturing images of a movement or position of the object, a respiration measurer (not shown) for measuring the respiration of the object, an ECG measurer for measuring the electrical activity of the heart, or a temperature measurer for measuring a temperature of the object and transmit the acquired monitoring information to the controller 110. The controller 110 may in turn control an operation of the scanner 50 based on the monitoring information. Operations of the controller 110 will now be described in more detail.

The controller 110 may control overall operations of the scanner 50.

The controller 110 may control a sequence of signals formed in the scanner 50. The controller 110 may control the gradient magnetic field generator 52 and the RF coil unit 53 according to a pulse sequence received from the operating unit 10 or a designed pulse sequence.

A pulse sequence may include all pieces of information required to control the gradient magnetic field generator 52 and the RF coil unit 53. For example, the pulse sequence may include information about a strength, a duration, and application timing of a pulse signal applied to the gradient magnetic field generator 52.

The controller 110 may control a waveform generator (not shown) for generating a gradient wave, i.e., an electrical pulse according to a pulse sequence and a gradient amplifier (not shown) for amplifying the generated electrical pulse and transmitting the same to the gradient magnetic field generator 52. Thus, the controller 110 may control formation of a gradient magnetic field by the gradient magnetic field generator 52.

Furthermore, the controller 110 may control an operation of the RF coil unit 53. For example, the controller 110 may supply an RF pulse having a resonance frequency to the RF coil unit 30 that emits an RF signal toward the object, and receive an MR signal received by the RF control unit 53. In this case, the controller 110 may adjust emission of an RF signal and reception of an MR signal according to an operating mode by controlling an operation of a switch (e.g., a T/R switch) for adjusting transmitting and receiving directions of the RF signal and the MR signal based on a control signal.

The controller 110 may control a movement of the table 55 where the object is placed. Before MRI is performed, the controller 110 may move the table 55 according to which region of the object is to be imaged.

The controller 110 may also control the display 56. For example, the controller 110 control the on/off state of the display 56 or a screen to be output on the display 56 according to a control signal.

The controller 110 may be formed as an algorithm for controlling operations of the components in the MRI system 1, a memory (not shown) for storing data in the form of a program, and a processor for performing the above-described operations by using the data stored in the memory. In this case, the memory and the processor may be implemented as separate chips. Alternatively, the memory and processor may be incorporated into a single chip.

The operating unit 10 may control overall operations of the MRI system 1 and include an image processor 120, an input device 12, and an output device 13.

The image processor 120 may control the memory to store an MR signal received from the controller 110, and generate image data with respect to the object from the stored MR signal by applying an image reconstruction technique.

For example, if a k space (for example, also referred to as a Fourier space or a frequency space) of the memory is filled with digital data to complete k-space data, the image processor 120 may reconstruct image data from the k-space data by applying various image reconstruction techniques (e.g., by performing inverse Fourier transform on the k-space data).

Furthermore, the image processor 120 may perform various signal processing operations on MR signals in parallel. For example, the image processor 62 may perform signal processing on a plurality of MR signals received via a multi-channel RF coil in parallel so as to convert the plurality MR signals into image data. In addition, the image processor 120 may store not only the image data in the memory, or the controller 110 may store the same in an external server via a communication unit 60 as will be described below.

The input device 12 may receive, from the user, a control command for controlling the overall operations of the MRI system 1. For example, the input device 12 may receive, from the user, object information, parameter information, a scan condition, and information about a pulse sequence. The input device 12 may be a keyboard, a mouse, a track ball, a voice recognizer, a gesture recognizer, a touch screen in the display, or any other input device.

The output device 13 may output image data generated by the image processor 120. The output device 13 may also output a user interface (UI) configured so that the user may receive a control command related to the MRI system 1. The output device 13 may be a speaker, a printer, a display including a touch screen, or any other output device.

Furthermore, although FIG. 10 shows that the operating unit 10 and the controller 110 are separate components, the operating unit 10 and the controller 110 may be included in a single device as described above. Furthermore, processes respectively performed by the operating unit 10 and the controller 110 may be performed by another component. For example, the image processor 120 may convert an MR signal received from the controller 110 into a digital signal, or the controller 110 may directly perform the conversion of the MR signal into the digital signal.

The MRI system 1 may further include a communication unit 60 and be connected to an external device (not shown) such as a server, a medical apparatus, and a portable device (e.g., a smartphone, a tablet PC, a wearable device, etc.) via the communication unit 60.

The communication unit 60 may include at least one component that enables communication with an external device. For example, the communication unit 60 may include at least one of a local area communication module (not shown), a wired communication module 61, and a wireless communication module 62.

The communication unit 60 may receive a control signal and data from an external device and transmit the received control signal to the controller 110 so that the controller 110 may control the MRI system 1 according to the received signal.

Alternatively, by transmitting a control signal to an external device via the communication unit 60, the controller 110 may control the external device according to the control signal.

For example, the external device may process data according to a control signal received from the controller 110 via the communication unit 60.

A program for controlling the MRI system 1 may be installed on the external device and may include instructions for performing some or all of the operations of the controller 110.

The program may be preinstalled on the external device, or a user of the external device may download the program from a server providing an application for installation. The server providing an application may include a recording medium having the program recorded thereon.

Embodiments may be implemented through non-transitory computer-readable recording media having recorded thereon computer-executable instructions and data. The instructions may be stored in the form of program codes, and when executed by a processor, generate a predetermined program module to perform a specific operation. Furthermore, when being executed by the processor, the instructions may perform specific operations according to the embodiments.

While one or more embodiments have been described with reference to the figures, it will be understood by those of ordinary skill in the art that various changes in form and details may be made therein without departing from the spirit and scope of the present disclosure as defined by the following claims. Accordingly, the above embodiments and all aspects thereof are examples only and are not limiting.

**Claims**

1. A magnetic resonance imaging (MRI) apparatus comprising:

   a controller configured to acquire, based on a gradient echo sequence, an MR signal from an object; and
   an image processor configured to acquire a first image corresponding to a time point when the MR signal has a largest phase variation, acquire a second image corresponding to a time point when the MR signal has a smallest phase variation, and obtain an arterial image including an artery of the object by using the first image and the second image.

2. The MRI apparatus of claim 1, wherein the phase variation corresponds to a velocity of blood flow in the object.

3. The MRI apparatus of claim 1, wherein the gradient echo sequence comprises a velocity-selective excitation block, and
   the velocity-selective excitation block sequentially comprises a first radio frequency (RF) pulse having a first flip angle, a velocity encoding gradient, and a second RF pulse having a second flip angle which has a same magnitude as that of the first flip angle and has a different direction than that of the first flip angle.

4. The MRI apparatus of claim 1, wherein the image processor is further configured to acquire k-space data based on the MR signal along a radial trajectory.

5. The MRI apparatus of claim 4, wherein the image processor is further configured to reconstruct a plurality of undersampled images respectively corresponding to different time points based on the acquired k-space data.

6. The MRI apparatus of claim 5, wherein the image processor is further configured to determine, based on signal intensities of the artery in the plurality of undersampled images, the first image and the second image from the plurality of undersampled images.

7. The MRI apparatus of claim 1, wherein the image processor is further configured to obtain the arterial image based on a difference between intensities of image signals respectively corresponding to the first and second images.

8. The MRI apparatus of claim 1, wherein the image processor is further configured to acquire a plurality of first images and a plurality of second images, obtain a first composite image by combining the acquired plurality of first images, and obtain a second composite image by combining the acquired plurality of second images.

9. The MRI apparatus of claim 1, wherein the controller is further configured to acquire a signal corresponding to an electrical activity of a heart based on the phase variation.

10. The MRI apparatus of claim 1, wherein the first image corresponds to a systole phase of a heart of the object, and the second image corresponds to a diastole phase of the heart of the object.

11. The MRI apparatus of claim 1, wherein the first image and the second image each include the artery and a vein of the object.

12. A method of obtaining a magnetic resonance (MR) image, the method comprising:

acquiring an MR signal from an object based on a gradient echo sequence;
acquiring a first image corresponding to a time point when the MR signal has a largest phase variation and a second image corresponding to a time point when the MR signal has a smallest phase variation; and
obtaining an arterial image including an artery of the object by using the first image and the second image.

13. The method of claim 12, wherein the phase variation corresponds to a velocity of blood flow in the object.

14. The method of claim 12, wherein the gradient echo sequence comprises a velocity-selective excitation block, and the velocity-selective excitation block sequentially comprises a first radio frequency (RF) pulse having a first flip angle, a velocity encoding gradient, and a second RF pulse having a second flip angle, which has a same magnitude as that of the first flip angle and has a different direction than that of the first flip angle.

15. A non-transitory computer-readable recording medium having recorded thereon a program which, when executed by a computer system, causes the computer system to execute the method of claim 12.

# FIG. 1

100

```
┌─────────────────────────────────┐
│                         110      │
│   ┌─────────────────────┐        │
│   │     CONTROLLER      │        │
│   └─────────────────────┘        │
│              │                   │
│              ▼          120      │
│   ┌─────────────────────┐        │
│   │   IMAGE PROCESSOR   │        │
│   └─────────────────────┘        │
│                                  │
└─────────────────────────────────┘
```

# FIG. 2

201

205   203

Static tissue

Blood

# FIG. 3

Imaging slice:
Saturation pulse

Venous spines:
Saturation pulse

Fat saturation pulse

310

301

303

305

bSSFP acquisition    320

R-spike

Q1

330

# FIG. 4A

FIG. 4B

$\phi = \gamma G(T/2)_\nu + \phi_0$

EP 3 168 636 A2

# FIG. 5A

# FIG. 5B

# FIG. 6

| | |
|---|---|
| VEIN | |
| ARTERY | |

# FIG. 7

# FIG. 8

# FIG. 9

START

↓

ACQUIRE MR SIGNAL FROM OBJECT BASED ON GRADIENT ECHO SEQUENCE —— S110

↓

ACQUIRE FIRST IMAGE CORRESPONDING TO TIME POINT WHEN MR SIGNAL HAS LARGEST PHASE VARIATION AND SECOND IMAGE SIGNAL HAS LARGEST PHASE VARIATION AND SECOND IMAGE VARIATION —— S120

↓

OBTAIN ARTERIAL IMAGE INCLUDING ARTERY OF OBJECT BY USING FIRST AND SECOND IMAGES —— S130

↓

END

# FIG. 10

EP 3 168 636 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020150157487

- KR 1020160086408